# FASCICULE DE BREVET EUROPEEN

(11) **EP 1 685 156 B1**
(45) Date de publication et mention de la délivrance du brevet: **07.04.2010**
(21) Numéro de dépôt: 04805492.8
(22) Date de dépôt: 19.11.2004
(51) Int. Cl.: C07K 14/44

(54) **NOUVEAUX MOYENS POUR LA PRÉVENTION DES LEISHMANIOSES**
NEUE MITTEL ZUR PRÄVENTION VON LEISHMANIOSE
NOVEL AGENTS FOR THE PREVENTION OF LEISHMANIOSIS

(30) Priorité: 19.11.2003 FR 0313555; 25.06.2004 FR 0407010
(43) Date de publication de la demande: 02.08.2006
(73) Titulaire: Institut de Recherche pour le Développement ( IRD), 75480 Paris Cedex 10 (FR)
(72) Inventeur: LEMESRE, Jean-Loup, F-34070 Montpellier (FR); CAVALEYRA, Mireille, F-34000 Montpellier (FR); SERENO, Denis, F-34560 Poussan (FR); HOLZMULLER, Philippe, F-34560 Poussan (FR)
(74) Mandataire: Peaucelle, Chantal
(86) Numéro de dépôt international: PCT/FR2004/002955
(87) Numéro de publication internationale: WO 2005/051989

(56) Documents cités:
- WO-A-94/26899
- LOHMAN K L ET AL: "Molecular cloning and characterization of the immunologically protective surface glycoprotein GP46/M-2 of Leishmania amazonensis" PROCEEDINGS OF THE NATIONAL ACADEMY OF SCIENCES OF USA, NATIONAL ACADEMY OF SCIENCE. WASHINGTON, US, vol. 87, novembre 1990 (1990-11), pages 8393-8397, XP002204079 ISSN: 0027-8424
- DUMONTEIL E ET AL: "DNA vaccines induce partial protection against Leishmania mexicana" VACCINE, BUTTERWORTH SCIENTIFIC. GUILDFORD, GB, vol. 21, no. 17-18, 16 mai 2003 (2003-05-16), pages 2170-2177, XP004421134 ISSN: 0264-410X
- HANDMAN E ET AL: "Therapy of murine cutaneous leishmaniasis by DNA vaccination" VACCINE, BUTTERWORTH SCIENTIFIC. GUILDFORD, GB, vol. 18, no. 26, juillet 2000 (2000-07), pages 3011-3017, XP004199096 ISSN: 0264-410X
- LEBOWITZ J H ET AL: "DEVELOPMENT OF A STABLE LEISHMANIA EXPRESSION VECTOR AND APPLICATION TO THE STUDY OF PARASITE SURFACE ANTIGEN GENES" PROCEEDINGS OF THE NATIONAL ACADEMY OF SCIENCES OF USA, NATIONAL ACADEMY OF SCIENCE. WASHINGTON, US, vol. 87, décembre 1990 (1990-12), pages 9736-9740, XP002052133 ISSN: 0027-8424
- KIMA P E ET AL: "Presentation via the class I pathway by Leishmania amazonensis-infected macrophages of an endogenous leishmanial antigen to CD8+ T cells." JOURNAL OF IMMUNOLOGY (BALTIMORE, MD. : 1950) 15 AUG 1997, vol. 159, no. 4, 15 août 1997 (1997-08-15), pages 1828-1834, XP002336360 ISSN: 0022-1767
- JIMENEZ-RUIZ A ET AL: "CLONING SEQUENCING AND EXPRESSION OF THE PSA GENES FROM LEISHMANIA INFANTUM" EUROPEAN JOURNAL OF BIOCHEMISTRY, BERLIN, DE, vol. 251, no. 1/2, 15 janvier 1998 (1998-01-15), pages 389-397, XP001159173 ISSN: 0014-2956
- MURRAY P J ET AL: "VARIANTS OF A LEISHMANIA SURFACE ANTIGEN DERIVED FROM A MULTIGENIC FAMILY" JOURNAL OF BIOLOGICAL CHEMISTRY, vol. 266, no. 36, 1991, pages 24477-24484, XP002296789 ISSN: 0021-9258
- SYMONS FIONA M ET AL: "Characterization of a polymorphic family of integral membrane proteins in promastigotes of different Leishmania species" MOLECULAR AND BIOCHEMICAL PARASITOLOGY, vol. 67, no. 1, 1994, pages 103-113, XP002336361 ISSN: 0166-6851
- BEETHAM JEFFREY K ET AL: "Glycoprotein 46 mRNA abundance is post-transcriptionally regulated during development of Leishmania chagasi promastigotes to an infectious form" JOURNAL OF BIOLOGICAL CHEMISTRY, vol. 272, no. 28, 1997, pages 17360-17366, XP002296788 ISSN: 0021-9258

## Description

L'invention a pour objet de nouveaux moyens pour la prévention des *leishmanioses* chez l'animal et chez l'homme.

Elle vise en particulier des molécules d'acides nucléiques codant pour des facteurs de virulence ou de pathogénicité chez *Leishmania* et leur utilisation pour produire de tels facteurs afin d'élaborer des compositions vaccinales contre les *leishmanioses.*

Les *leishmanioses* représentent l'une des six maladies parasitaires majeures et sont considérées, à ce titre, comme prioritaires par l'Organisation Mondiale de la Santé (O.M.S). Les leishmanies existent sous la forme promastigote extracellulaire, à l'intérieur du tube digestif de l'insecte vecteur (le phlébotome), et sous la forme amastigote intracellulaire, chez l'hôte mammifère. Plusieurs molécules, dont les lypophosphoglycanes (LPG) ou une métalloprotéase appelée gp63, semblent jouer un rôle important dans le pouvoir infectieux et la pathogénicité du parasite. Plus récemment, une famille de glycoprotéines appelées antigènes de surface de promastigotes, PSA (pour "Promastigote Surface Antigens"), a suscité un intérêt nouveau. Ces PSA sont caractérisées par la présence de motifs répétés riches en leucine pouvant intervenir dans les interactions de type protéine/protéine et confèrent une immunité protectrice à médiation cellulaire de type Th 1 chez la souris. Chez des organismes, tels que les bactéries ou les plantes, il est apparu que les PSA étaient impliquées dans des fonctions comme l'adhésion cellulaire, la résistance aux pathogènes et la transduction de signaux.

Cependant, aucun rôle biologique n'a été décrit, ni suggéré, chez *Leishmania.*

Ce rôle a pu être étudié par les inventeurs grâce à la technique qu'ils détiennent pour cultiver des promastigotes et des amastigotes de *Leishmania* dans des conditions asériques et axéniques , avec un milieu totalement défini , c-à-d dont les constituants sont tous identifiés, et qui fait l'objet du brevet FR 93 05 779 du 13 mai 1993 au nom de l'IRD (ex. ORSTOM). La maîtrise de ce procédé leur permet de disposer de formes parasitaires dépourvues des contaminants apportés jusqu'alors par les milieux de culture, et de déterminants antigéniques sous une forme hautement purifiée.

Dans ledit brevet FR de la Demanderesse, on a déjà décrit l'isolement et l'identification d'une PSA excrétée/secrétée (antigène d'excrétion/secrétion ou AES en abrégé) de 38 kDa et de 45 kDa dans le surnageant de culture de *L. amazoniensis.*

Les inventeurs ont à présent isolé et cloné l'ADNc codant pour cette protéine et procédé à l'évaluation de son rôle dans la biologie du parasite en mettant au point une stratégie de transgénèse additionnelle. Ces travaux ont permis de mettre en évidence l'implication de cette PSA en tant que facteur de virulence et/ou de pathogénicité et d'élaborer des constructions permettant de surexprimer le gène de *Leishmania* codant pour cette PSA, ce qui permet de développer des moyens pour l'obtention de compositions vaccinales contre les *leishmanioses.*

L'invention a donc pour but de fournir des séquences d'acides nucléiques capables de coder pour des PSA de formes promastigotes et de formes amastigotes de *Leishmania,* constituant des facteurs de virulence et/ou de pathogénicité.

Elle vise tout particulièrement à fournir des vecteurs de surexpression de ces PSA, ainsi que des parasites génétiquement modifiés.

L'invention vise en outre les surnageants des milieux de culture des PSA obtenues, ainsi que les PSA isolées, purifiées, et la mise à profit de leurs propriétés pour élaborer des compositions vaccinales contre les *leishmaniose.*

Les séquences d'acides nucléiques de l'invention correspondent à des acides nucléiques isolés capables de coder pour une PSA de formes promastigotes ou de formes amastigotes de *Leishmania,* lesdits acides nucléiques répondant à l'une des séquences SEQ ID N°1, SEQ ID N°2, SEQ ID N°4, SEQ ID N°5 et SEQ ID N°11 et codant pour des PSA de séquences, respectivement, SEQ ID N°6, SEQ ID N°7, SEQ ID N°9, SEQ ID N°10 et SEQ ID N°12.

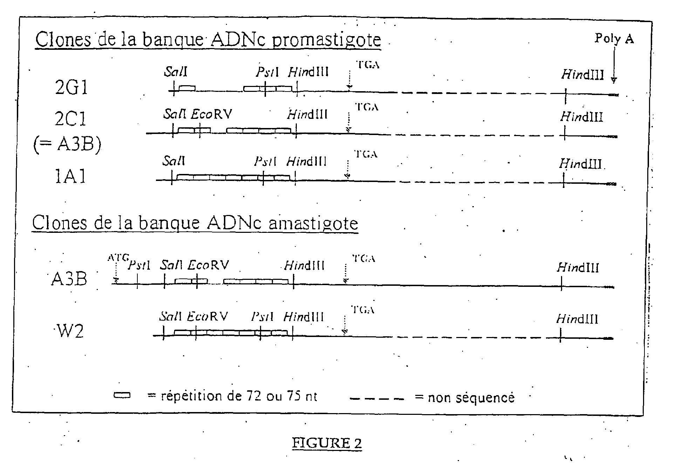

Les séquences d'acides nucléiques de l'invention sont plus spécialement des séquences de clones d'ADNc appartenant à une famille répondant aux caractéristiques illustrées par la figure 2 et comprenant en particulier un site de restriction *Sal*I et deux sites de restriction *Hind*III, avec un codon stop situé en aval du premier site *Hind*III.

L'invention vise en particulier les clones d'ADNc de ladite famille comportant un site de restriction *Eco*RV et/ou *Pst*I entre les deux sites *Sal*I et *Hind*III, ou de part et d'autre du site *Sal*I.

L'invention vise également les protéines immunogènes isolées, caractérisées en ce qu'elles présentent une séquence telle que codée par les acides nucléiques définis ci-dessus. Elle vise en particulier les protéines répondant aux séquences SEQ ID N°6, SEQ ID N°7, SEQ ID N°9, SEQ ID N°10 ou SEQ ID N°12.

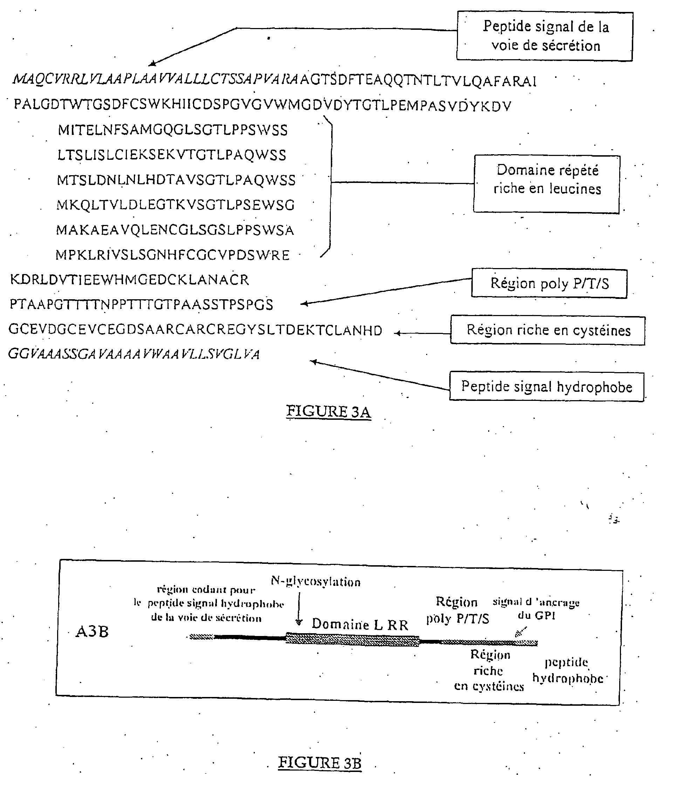

Ces protéines appartiennent à la famille dite des antigènes de surface de promastigotes (PSA en abrégé) et possèdent des régions caractéristiques illustrées sur les figures 3A et 3B. Ces protéines peuvent être modifiées post-traductionellement par des N-glycosylations, phosphorylations et l'ancrage d'un GPI. Elles possèdent un peptide signal hydrophobe en position carboxy-terminale.

Par clonage directionnel des séquences définies ci-dessus dans un vecteur d'expression, les inventeurs ont obtenu des constructions permettant de les exprimer en position sens.

L'invention vise donc des constructions d'acides nucléiques, caractérisées en ce qu'elles comportent des acides nucléiques isolés en position sens, capables de coder pour une protéine immunogène de formes promastigotes ou de formes amastigotes de *Leishmania*, ces protéines répondant à l'une des séquences SEQ ID N°6, SEQ ID N°7, SEQ ID N°9, SEQ ID N°10 et SEQ ID N°12.

L'invention vise notamment les constructions d'acides nucléiques comportant des séquences de clones d'ADNc appartenant à une famille répondant aux caractéristiques illustrées par la figure 2 et comprenant en particulier un site de restriction *Sal*I et deux sites de restriction *Hind*III, avec un codon stop situé en aval du premier site *Hind*III.

Les clones d'ADNc comportant un site de restriction *Eco*RV et/ou *Pst*I entre les deux sites *Sal*I et *Hind*III, ou de part et d'autre du site *Sal*I sont particulièrement préférés.

Des constructions particulièrement avantageuses comportent comme séquences d'acides nucléiques, une séquence choisie parmi SEQ ID N°1, SEQ ID N°2, SEQ ID N°4, SEQ ID N°5 et SEQ ID N°11, ces séquences codant respectivement pour des protéines de séquences SEQ ID N°6, SEQ ID N°7, SEQ ID N°9, SEQ ID N°10 et SEQ ID N°12.

Des constructions préférées comportent lesdites séquences d'acides nucléiques dans un plasmide à multiplication rapide tel que pTex.

Elle vise également les souches de *Leishmania* transfectées par de telles constructions qu'il s'agisse de formes promastigotes, ou de formes amastigotes.

Des souches transfectées préférées, compte tenu des applications vaccinales visées, sont des souches de *L.infantum.*

De manière avantageuse les PSA sont produites en grande quantité, de manière constitutive, dans les parasites.

L'invention vise également un procédé de transfection d'un parasite de *Leishmania,* caractérisé en ce qu'on introduit dans le parasite de *Leishmania* un vecteur tel que défini plus haut, comportant un marqueur, on sélectionne les parasites transfectés grâce audit marqueur, on les met en culture dans un milieu axénique et asérique totalement défini et on récupère le surnageant de culture qui renferme les protéines immunogènes présentes dans des concentrations de l'ordre de 10 à 20 fois supérieures à celle produite par la souche mère de *Leishmania.*

L'introduction du vecteur dans le parasite est par exemple réalisée par électroporation.

L'insertion de ces acides nucléiques dans les parasites permet d'augmenter le pouvoir infectieux de ces derniers : leur capacité à survivre dans le macrophage infecté et à s'y multiplier est supérieure jusqu'à 5 fois celle du parasite non transfecté par de tels acides nucléiques.

Lesdites PSA sont produites en grande quantité dans le surnageant du milieu de culture des parasites. L'invention vise donc également les surnageants des milieux de cultures desdits parasites génétiquement modifiés, ainsi que les PSA isolées à partir de ces surnageants et purifiées.

L'invention fournit ainsi des moyens de grand intérêt pour répondre à la demande industrielle de disposer de quantités importantes de protéines constituant des facteurs de virulence/pathogénicité chez les *Leishmanies.*

Grâce à leur pouvoir immunogène, ces protéines permettent d'obtenir, après immunisation d'animaux selon des techniques classiques, des anticorps polyclonaux et d'élaborer des anticorps monoclonaux. L'immunisation de souris a ainsi permis d'obtenir des anticorps anti Ig G2A et celle de chien des anticorps IgG2.

L'invention vise donc également de tels anticorps et mise à profit de leurs propriétés pour élaborer à une échelle exportable industriellement des compositions vaccinales contre les *leishmanies* chez l'homme ou animal.

Les applications diagnostiques de ces anticorps font également partie de l'invention.

D'autres caractéristiques et avantages de l'invention seront donnés dans les exemples qui suivent dans lesquels il sera fait référence aux figures 1 à 8, qui représentent, respectivement :
- la figure 1, l'alignement en 3' des séquences nucléotidiques de clones d'ADNc selon l'invention ;
- la figure 2, un schéma récapitulatif des séquences nucléotidiques des clones d'ADNc obtenus après criblage immunologique par un anticorps monoclonal anti-AES des banques d'expression des formes promastigotes et des formes amastigotes de *L. amazonensis.* Les sites des enzymes de restriction sont indiqués au-dessus de chaque séquence ;
- la figure 3A, la localisation de différentes régions protéiques, caractérisées par leur composition particulière en acides aminés, présentes sur la séquence protéique déduite de l'ADNc du clone A3B, et la figure 3B une représentation schématique de la séquence protéique déduite de l'ADNc du clone A3B codant pour une PSA ;
- la figure 4, les analyses des transcrits par RT-PCR chez les formes promastigotes (P) et amastigotes (A) ;
- la figure 5, le niveau de production de la protéine par western-blotting, à l'aide d'un anticorps anti-PSA,
- la figure 6, l'effet de la surexpression d'une PSA de *L. amazonensis* sur le pouvoir infectieux des parasites,
- la figure 7, les séquences nucléotidiques SEQ ID N°1 à 5 et 11, respectivement des clones A3B, 2C1, 1A1, 2G1, W2, de promastigotes et d'amastigotes de *L.amazonensis* et IJ11 de promastigotes de *L.infantum,* et les séquences d'acides aminés codées correspondantes SEQ ID N°6 à 10 et 12, et
- la figure 8, l'indice parasitaire déterminé au cours de l'infection *in vitro* de macrophages canins par une souche sauvage ou des clones sélectionnés de promastigotes de *L.infantum* à différents temps d'incubation.

### 1 - Caractérisation moléculaire des immunogènes majeurs des AES des formes promastigotes et amastigotes de L. amazonensis. (Lma en abrégé)

Cette caractérisation a été effectuée en criblant des banques d'expression d'ADNc des formes promastigotes et des formes amastigotes de *L. amazonensis* à l'aide d'un anticorps monoclonal dirigé contre l'immunogène majeur des AES.

### - Caractéristiques des banques d'ADNc :

Deux banques d'expression d'ADNc, respectivement de formes promastigotes, et de formes amastigotes de *L. amazonensis* ont été réalisées. Les caractéristiques de ces banques sont présentées au tableau I. Les parasites de phase exponentielle et stationnaire ont été mélangés afin d'avoir accès aux différents transcrits pouvant être exprimés au cours des différentes étapes de leur culture *in vitro.* 5 x 10⁴ phages par banque ont ensuite été criblés immunologiquement avec l'anticorps monoclonal F5 dilué au 1/500^{ème}. L'obtention de cet anticorps fait l'objet de l'exemple dans le brevet FR mentionné ci-dessus.

**Tableau I**

| Banque d'ADNc Lma LES J4 + J7 | Promastigotes | Amastigotes |
|---|---|---|
| récoltes J4 + J7 | 7,8.10⁹ | 7,8.10⁹ |
| Titration après packaging | 350 000 | 500 000 |
| Titre après amplification | 8,32.10⁷ ph/ul | 2,16.10⁸ ph/ul |

| | | |
|---|---|---|
| J4 + J7 = parasites récoltés au 4^{ème} jour, en phase exponentielle, et au 7^{ème} jour, en phase stationnaire de leur croissance. | | |

### - Isolement et séquençage des clones reconnus par l'anticorps monoclonal F5

13 clones de la banque promastigote et 11 clones se sont révélés positifs de la banque amastigote se sont révélés positifs. Tous ces clones ont été isolés par criblage secondaire et tertiaire.

L'ADN plasmidique de l'ensemble des clones isolés a été analysé après différentes digestions enzymatiques et les ADNc possédant des inserts de plus grande taille, par digestion *EcoRI*/*XhoI,* ont été retenus afin d'éliminer les ADNc trop tronqués en 5'. Comme le montre le tableau II, les clones 1A1, 1B1, 2B3, 2C1, 2D1 et 2E1 de la banque d'ADNc de promastigotes et les clones A3B, V4A, V5, W2 et W3 de la banque d'amastigotes présentent les inserts de plus grande taille.

L'analyse de ces clones, en déterminant la présence ou non de deux sites d'enzymes de restriction (*Hind*III et *Sal*I) préalablement sélectionnés, a montré qu'ils présentaient une forte homologie de leur séquence nucléotidique.

Par double digestion *Hind*III/*Sal*I, trois classes différentes de clones ont été mises en évidence avec un fragment *Hind*III/*Sa*II d'une taille, respectivement, inférieure à 400 pb (clone 2G1), de 500 pb (clones de type 2C1 et A3B) ou de 600 pb (clones de type 1A1 ou W2). Ainsi cinq types de clones, choisis selon les caractéristiques particulières de leur ADN (la taille de l'insert et la localisation de certains sites d'enzyme de restriction) sont représentés en caractère gras dans le tableau II.

**Tableau II Banque d'ADNc de promastigotes de Lma**

| Clones ADNc | **1A1** | 1B1 | 1C1 | 1D5 | 1F1 | 2A2 | 2B3 | **2C1** | 2D1 | 2E1 | 2F1 | **2G1** | B3 A |
|---|---|---|---|---|---|---|---|---|---|---|---|---|---|
| Taille des inserts EcoRI/Xhol (kb) | **2,5** | 2,5 | 2-2,2 | 0,5 | 2 | 2(>) | 2,5 | **2,4** | 2,4 | 2,4 | 2 | **1,7-2** | 1,7 |
| Carte de restriction | | | | | | | | | | | | | |
| *Sal*I | O | O | O | N | N | N | O | O | O | O | O | N | N |
| *Hind*III | 1,1 | 1,1 | 1,1 | / | 1,1 | 1,1 | 1,1 | 1,1 | 1,1 | 1,1 | 1,1 | 1,1 | 1,1 |
| *Hind*III/*Sal*I (pb) | **600** | 600 | 500 | N | N | N | 600 | **500** | 500 | 500 | N | **<400** | N |
| Expression de protéine recombinant e | | | | | | | | | | | | | |
| (kDa) | **45** | / | 40 | / | / | / | / | **42,5** | / | / | 39 | ? | **18** |

### Banque d'ADNc amastigotes de Lma

| Clones ADNc | **A3B** | V1B | V2D | V3A | V4A | V5 | W1A | W1C | **W2** | W3 | W5 |
|---|---|---|---|---|---|---|---|---|---|---|---|
| EcoRI/Xhol (kb) | **2,3** | 2-2,2 | 2,2 | ? | 2,3 | 2,3 | 2 | 2 | **2,3** | 2,2 | 1,7 |
| Carte de restriction | | | | | | | | | | | |
| *Sal*I | O | O | O | N | O | O | N | N | O | O | N |
| *Hind*III | 1,1 | 1,1 | 1,1 | 1,1 | 1,1 | 1,1 | 1,1 | 1,1 | 1,1 | 1,1 | 1,1 |
| *HindIII*/ *SalI* (pb) | **505** | 500 | 500 | N | 500 | 500 | N | N | **600** | 500< | N |
| Expression de protéine recombinante | | | | | | | | | | | |
| (kDa) | 42,5 | / | 36,5 | / | 36,5 | 43 | / | / | 45 | / | / |

| | | | | | | | | | | | |
|---|---|---|---|---|---|---|---|---|---|---|---|
| O= oui, pour site présent ; N= non, pour site absent ; / = réalisé ; ? = résultat non obtenu. | | | | | | | | | | | |

Le tableau II donne également les résultats relatifs à la capacité des clones à exprimer une protéine recombinante. L'IPTG a été utilisé comme agent d'induction. Les échantillons ont été analysés par SDS-PAGE et immunoblot en présence de l'anticorps anti-AES des formes promastigotes et/ou des formes amastigotes, préalablement absorbé en présence de lysat de *E. coli.* On obtient des résultats équivalents. On note pour les clones d'intérêt l'expression de différentes protéines recombinantes allant de 42,5 kDa de poids moléculaire apparent (clone 2C1), à 43 kDa (clone A3B) ou 45 kDa (1A1 et W2).

On rapporte sur le document "Listage des séquences", les résultats du séquençage:
- des trois types de clones suivants de la banque promastigote :
   le clone de type 1A1 (SEQ ID N°3), qui exprime une protéine de séquences SEQ ID N°8 de plus grand poids moléculaire. Les clones de type 1B1 et 2B3 sont du même type que ce clone ;
   . le clone 2C1 (SEQ ID N°2), qui exprime une protéine recombinante de poids moléculaire inférieur à celle du clone 1A1, présentant une séquence SEQ ID N°7.
   . le clone 2G1 (SEQ ID N°4), qui a la particularité de posséder un petit fragment *Hind*III/*Sal*I*,* qui exprime une protéine recombinante de poids moléculaire inférieur à celle du clone 1A1, présentant une séquence SEQ ID N°9.
- des deux clones suivants de la banque amastigote :
   les clones dé type A3B (SEQ ID N°1), qui expriment une protéine recombinante d'environ 43 kDa, de séquence SEQ ID N°6 et possèdent un fragment *Hind*III/ *Sal*I de 500 pb, le clone V5 apparaissant identique. Les clones V2D et V4A sont considérés comme des clones tronqués du même type ;
   . le clone W2 (SEQ ID N°5), qui exprime une protéine recombinante de 45 kDa, de séquence SEQ ID N°10 et qui présente un fragment *Hind*III/ *Sal*I de 600 pb.

### . Etude des cinq séquences d'ADNc

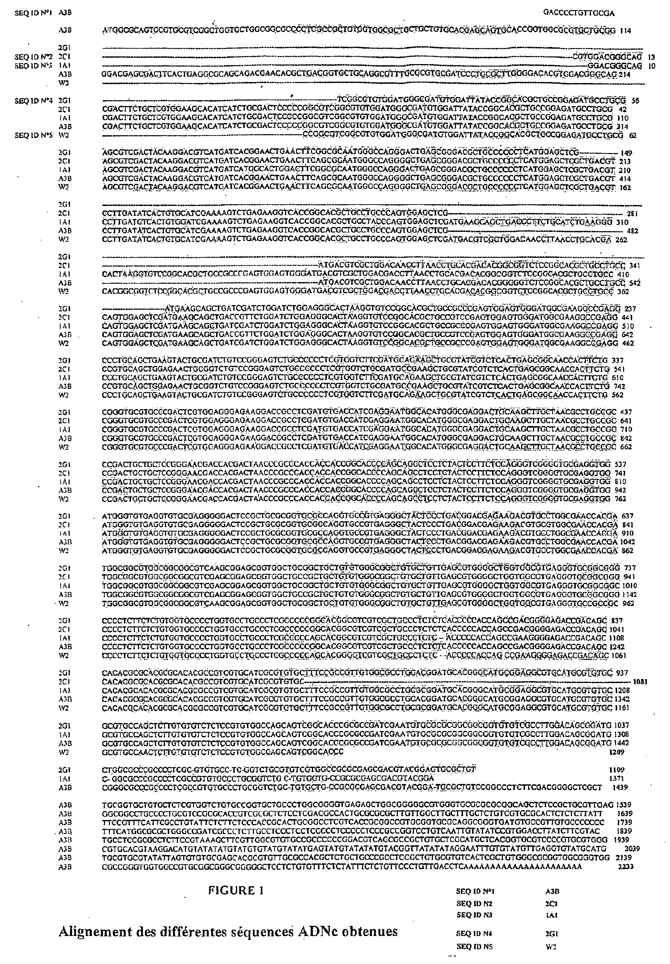

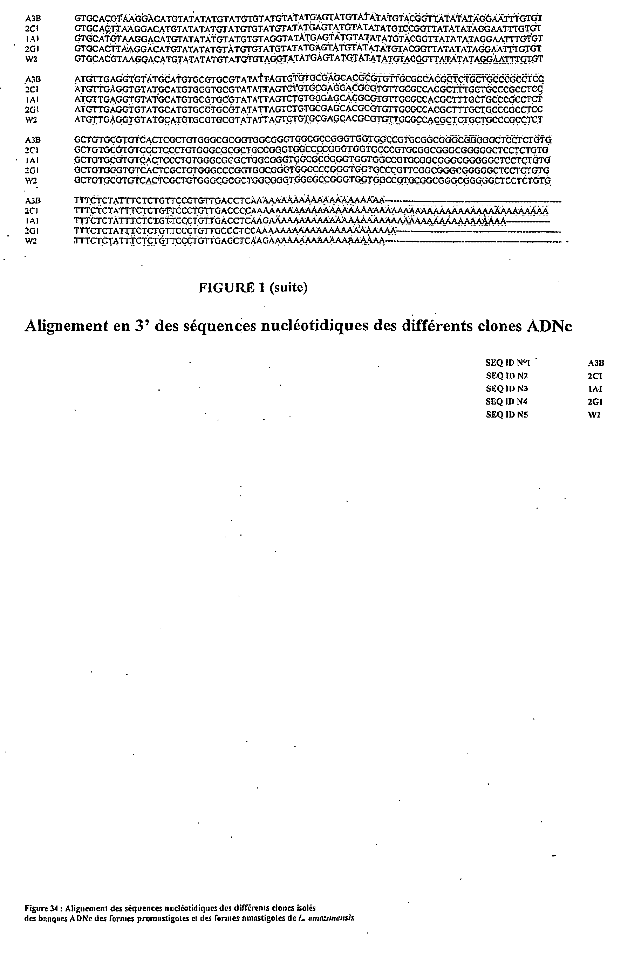

L'alignement des cinq séquences d'ADNc obtenues est représenté à la figure 1 où les différences entre ces clones sont seulement dues à la présence d'une séquence tronquée en 5' et/ou l'insertion de séquences d'environ 72 nucléotides du côté 5'. Les clones présentent ainsi une (clones 2C1 et A3B) ou trois (clones 1A1 et W2) insertions. Hormis ces zones d'insertions, les clones présentent des homologies de l'ordre de 99% et peuvent être considérés comme appartenant à une famille d'ADNc. Seul le clone A3B possède le codon ATG d'initiation, les autres clones étant tronqués en 5'. Cependant l'ADNc A3B ne possède pas la séquence de 39 nt codant pour le "splice leader" retrouvée en 5' sur tous les ARNm de *Leishmania.*

Les ADNc des clones A3B et 2C1 présentent une homologie quasi totale et sont donc considérés comme identiques, l'ADNc du clone 2C1 correspondant à une partie tronquée en 5' de l'ADNc du clone A3B.

Le clone A3B, représentatif de cette famille a fait l'objet d'un séquençage entier dans les deux sens.

On a rapporté sur la figure 2 les sites des enzymes de restrictions pour chacun de ces clones.

Les séquences SEQ ID N°1 à 5, correspondent respectivement à celles des ADNc de A3B, 2C1, 1A1, 2G1, W2.

### - Analyse des différentes séquences protéiques déduites

La traduction des différentes séquences d'ADNc en séquences protéiques a été réalisée en choisissant le cadre de lecture correspondant à celui suggéré par la position du codon d'initiation sur le plasmide pB-SK, dont la transcription est sous le contrôle du promoteur du gène *lacZ* soumise à l'induction par l'IPTG.

La protéine A3B présente les régions illustrées sur les figures 3A et 3B. En NH₂-terminal, on identifie un peptide hydrophobe, pouvant être clivé, qui est décrit dans la littérature comme un peptide signal de la voie sécrétion. On rencontre ensuite le domaine répété riche en leucine, au nombre de 6 répétitions pour le clone A3B. A une dizaine d'acides aminés de la fin de ce domaine se trouve une région riche en proline, thréonine et sérine, appelée ci-après région poly P/T/S. Cette région est suivie d'une région riche en cystéines, pouvant être impliquée dans des ponts disulfures. Enfin la séquence protéique se termine par un peptide signal hydrophobe.

Les ADNc des clones A3B et 2C1 présentent une homologie quasi totale et sont donc considérés comme identiques, l'ADNc du clone 2C1 correspondant à une partie tronquée en 5' de l'ADNc du clone A3B.

Le clone A3B, représentatif de cette famille, a fait l'objet d'un séquençage entier dans les deux sens.

On a rapporté sur la figure 2, les sites des enzymes de restriction pour chacun de ces clones Nt=nucléotides ; ATG=codon d'initiation ; TAG=codon stop.

L'analyse sur le banque de donnée PROSITE montre que la protéine A3B possède un site de N-glycosylation localisé à la fin de chaque domaine répété riche en leucine, et 12 sites potentiels de phosphorylations.

L'analyse de la localisation de cette protéine sur le serveur PSORT prédit une localisation cytoplasmique à 92%, ce qui indique que la protéine est soluble. Cette protéine est vraisemblablement ancrée à la surface par un Glycosyl Phosphatidyl Inositol ou GPI. Le peptide signal hydrophobe peut donc être clivé et permettre l'ancrage du GPI au niveau de l'asparagine (D).

Le poids moléculaire théorique de la protéine du clone A3B diffère d'environ 2,9 kDa avec celui des protéines 1A1 et W2, ce qui est en accord avec la différence de 2,5 kDa observée entre les protéines recombinantes correspondantes. Cette différence est due à la présence d'un nombre variable de répétitions riches en leucines ou LRR, chacune présentant aussi une composition en acides aminés particulière.

Les poids moléculaires apparents et théoriques des quatre types de PSA de l'invention sont rassemblés dans le tableau III suivant.

**Tableau III**

| Type de PSA | P.M. de la protéine recombinante | P.M. théorique (non tronquée) | P.M. sans peptide signal (3,2 kDa) |
|---|---|---|---|
| 4 LRR (2G1) | / | 33,5 kDa | 30,3 kDa |
| 6 LRR (A3B) | 42,5 kDa | 38,5 kDa | 35,3 kDa |
| 7 LRR (1A1 et W2) | 45 kDa | 41,4 kDa | 38,2 kDa |

### 2 - Obtention de parasites génétiquement modifiés :

Le clonage directionnel du gène LaPSA 38s dans le vecteur d'expression pTex a permis d'obtenir une construction capable d'exprimer le gène PSA en position sens. Le plasmide pTex-LaPSA 38s orientation sens et le vecteur pTex vide ont ensuite été électroporés dans la souche sauvage *Leishmania infantum* Mon 1 Clone 1, puis les parasites ont été sélectionnés par la génétycine.

L'étude a été réalisée sur des parasites de l'espèce *L. infantum* sauvages (Sau), ceux tranfectés par le pTex vide (pTex) et ceux transfectés par le pTex contenant la séquence nucléotidique d'intérêt (Sens).

### Caractérisation moléculaire :

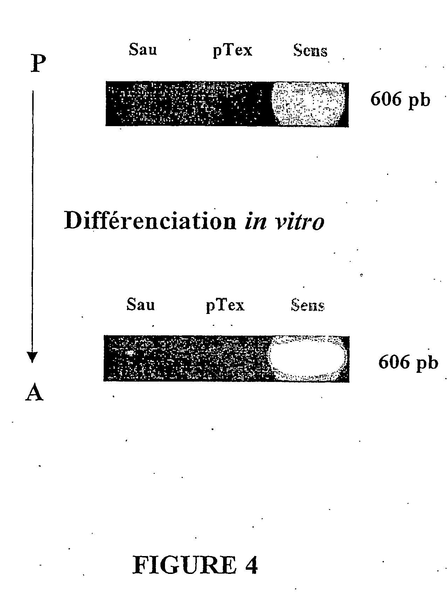

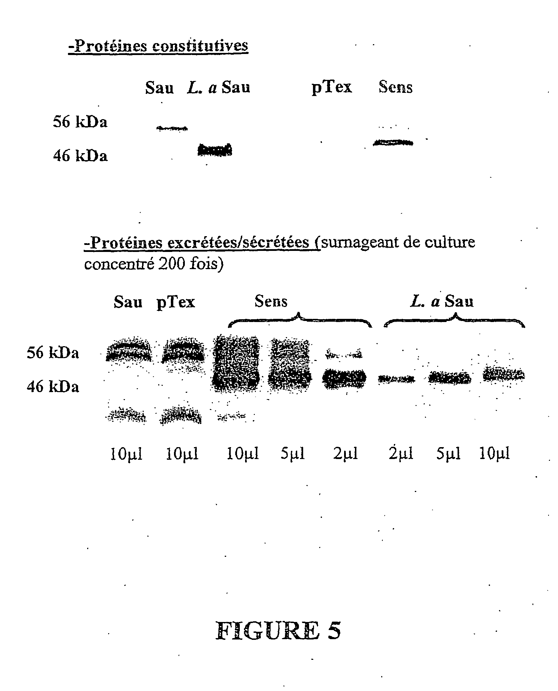

L'analyse de l'ADN total par southern blot montre que la construction sens est stable et amplifiée chez la souche transformée. Les résultats sont illustrés sur la figure 4 qui donne les analyses des transcrits par RT-PCR chez les deux formes, promastigotes (P) et amastigotes (A). La figure 5 donne le niveau de production de la protéine par western-blotting, à l'aide d'un anticorps anti-PSA (figure 5A : protéines constitutives ; figure 5B : protéines excrétées / sécrétées)

### Caractérisation phénotypique des mutants :

La comparaison des cinétiques de croissance entre Ldi WT, Ldi pTex et Ldi Sens montre que la surexpression de LaPSA 38s n'interfère pas avec la croissance des parasites. Seule une phase de latence plus longue est observée pour les souches transformées par rapport à la souche sauvage.

La sensibilité à la lyse par le complément humain a également été étudiée. Récemment, il a été démontré que la PSA de *L. amazoniensis* avait la propriété d'inhiber l'action du complément *in vitro.* Les promasigotes "sens" sont plus sensibles au complément. L'excès de PSA à la surface des parasites peut ainsi entraîner un clivage ainsi qu'une formation de complexes plus importante engendrant une lyse accrue.

### Etude de pouvoir infectieux des parasites.

Pour étudier l'effet de la surexpression de LaPSA 38s sur le pouvoir infectieux des parasites, la première approche a consisté à mettre en contact des promastigotes des souches transformées avec des macrophages de chien, qui est le réservoir domestique naturel de la *leishmaniose* viscérale.

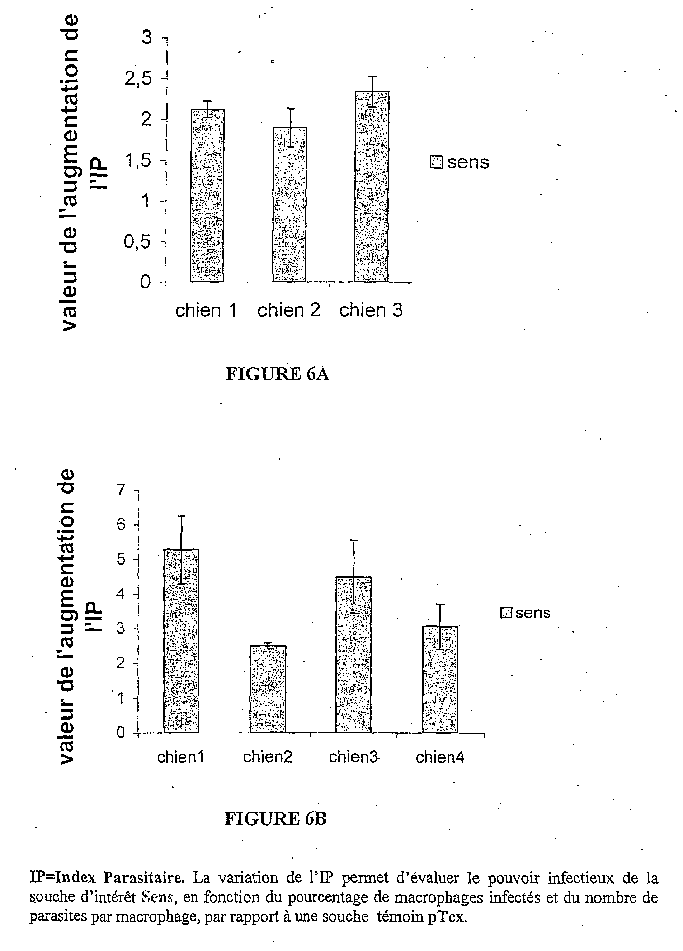

Les figures 6A et 6B donnent les résultats obtenus, respectivement, 2 h après contact avec les promastigotes et 48 h après contact avec les amastigotes sur ces figures, l'index parasitaire correspond au % de macrophages infectés par la souche Sens x le nombre de parasites par macrophage/% de macrophages infectés par la souche témoin (pTex) x le nombre de parasites par macrophage.

Les promastigotes surexprimant LaPSA 38s, présentent un pouvoir infectieux 2 fois plus important vis-à-vis des macrophages canins. De plus, après phagocytose, les amastigotes exprimant le transgène possèdent une capacité à survivre et à se multiplier dans la vacuole parasitophore significativement supérieure (2,5 à 5 fois) à celle du témoin transfecté par le vecteur vide.

### 2- Caractérisation moléculaire des AES de promastigote de L.infantum

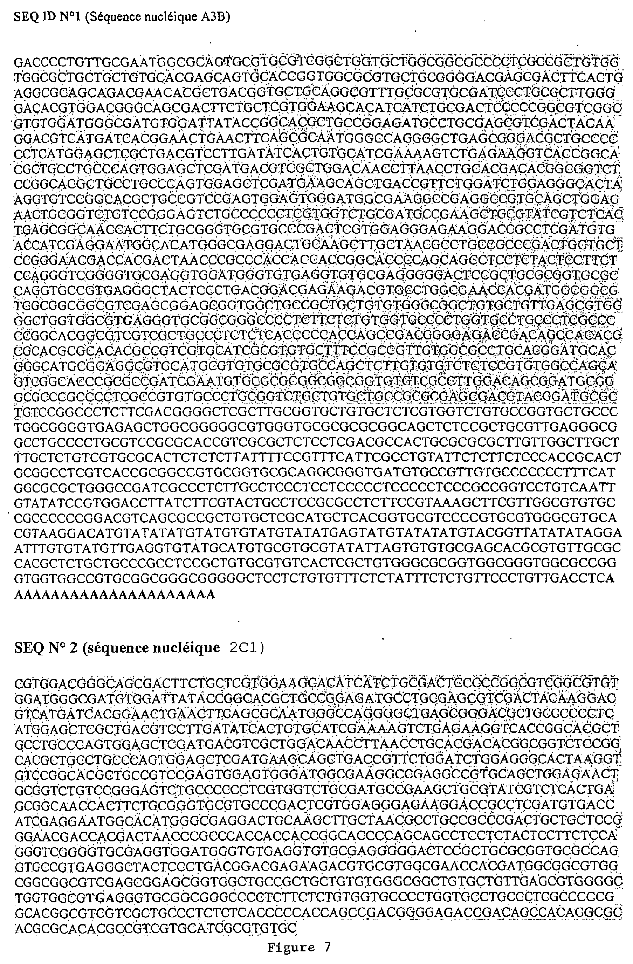

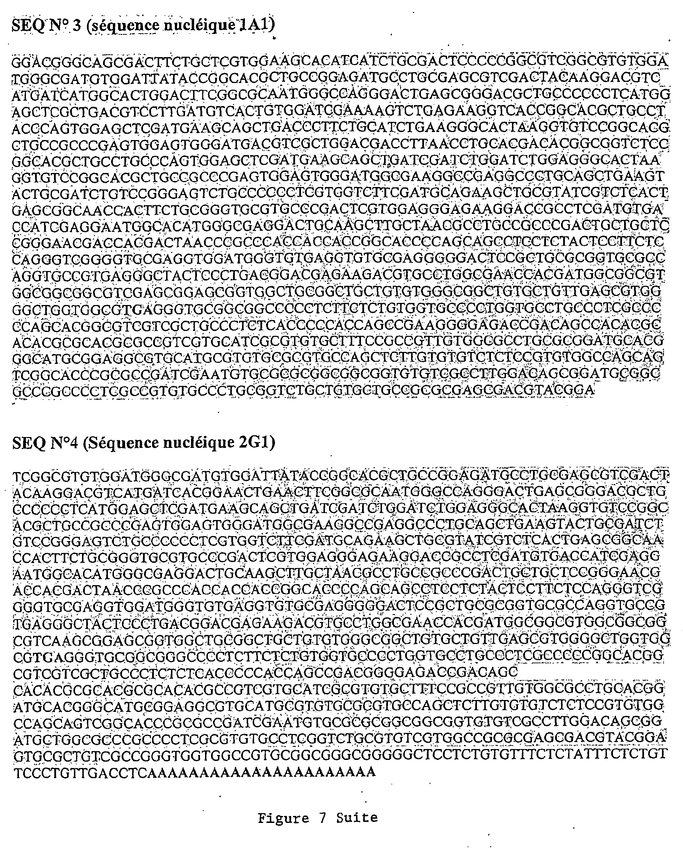

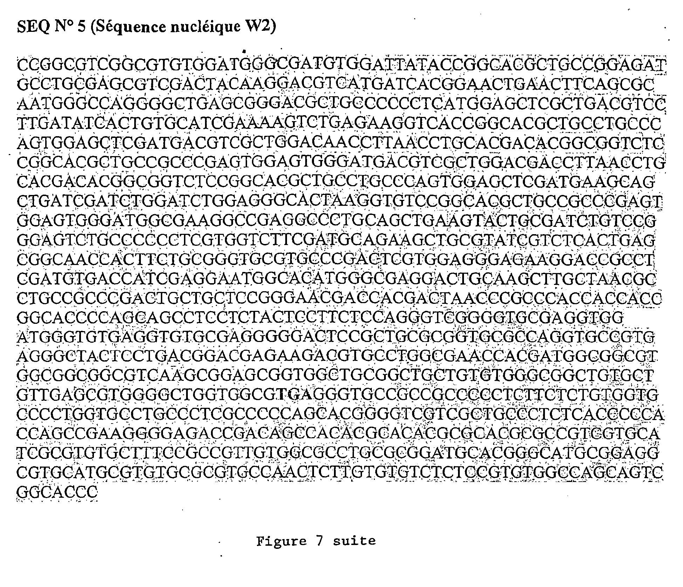

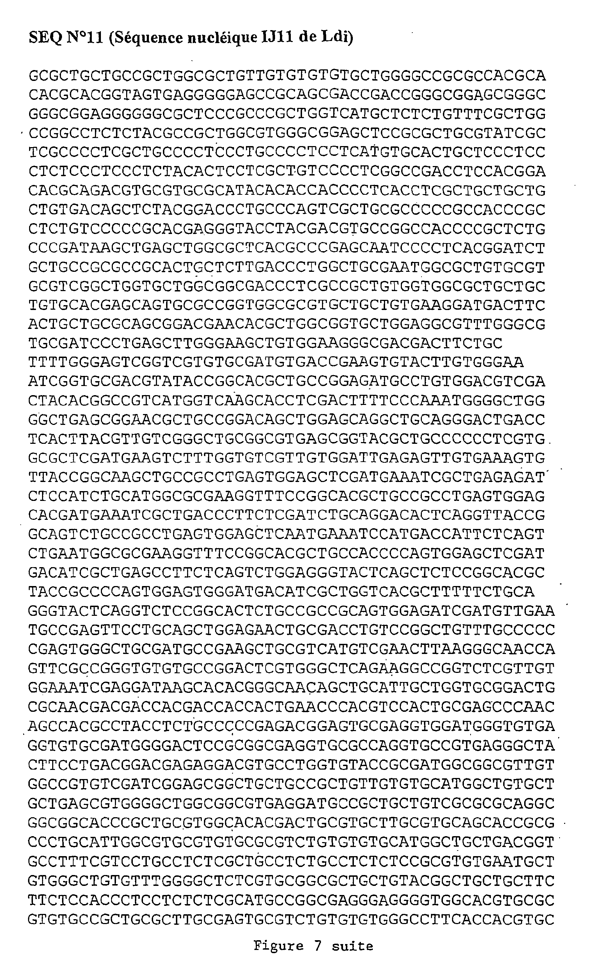

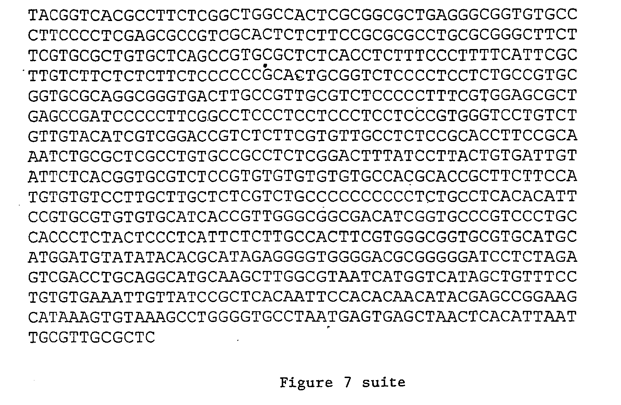

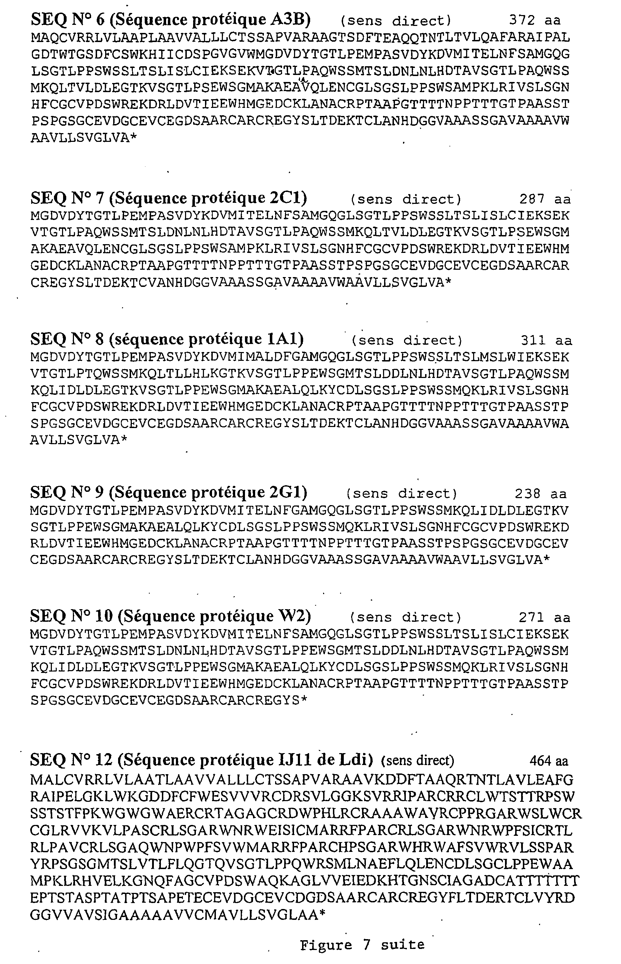

La séquence de nucléotides du clone IJ11 de promastigotes de *L.infantum* est donnée sur la figure 7 (SEQ ID N°11) ainsi que la séquence correspondante d'acides aminés (SEQ ID N°12).

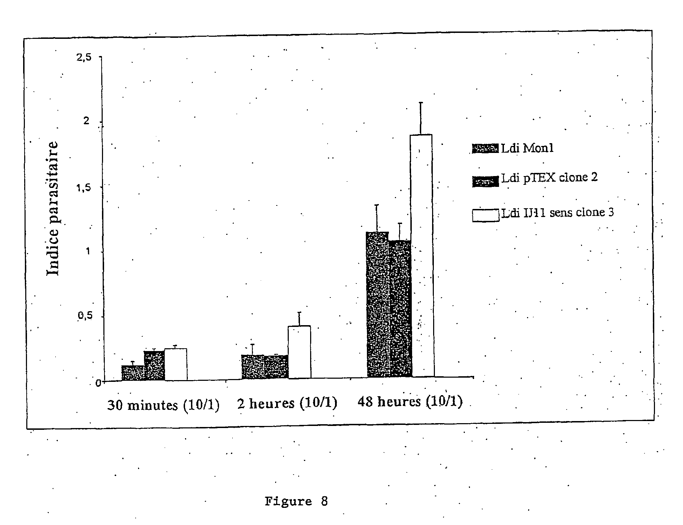

Sur la figure 8, on a rapporté l'indice parasitaire déterminé au cours de l'infection *in vitro* de macrophages canins par la souche sauvage ou les différents clones sélectionnés de formes promastigotes de *L.infantum* (MHON/MA/67/ITMAP-263, clone 2) à différents temps d'incubation. L'examen de ces résultats montre un attachement des parasites aux macrophages au bout de 30 min., une pénétration des parasites au bout de 2 heures et la survie et multiplication des amastigotes intracellulaires à 48 heures.

## Revendications

1. Constructions d'acides nucléiques, **caractérisées en ce qu'**elles comportent des acides nucléiques isolés en position sens, capables de coder pour une protéine immunogène de formes promastigotes ou de formes amastigotes de *Leishmania,* lesdits acides nucléiques répondant à l'une des séquences SEQ ID N°1, SEQ ID N°2, SEQ ID N°4, SEQ ID N°5 et SEQ ID N°11, ces séquences comprenant un site de restriction *Eco*RV entre les deux sites *Sal*I et *Hind*III, ou de part et d'autre du site *Sal*I.

2. Constructions selon la revendication 1, **caractérisées en ce que** lesdites séquences d'acides nucléiques sont clonées en position sens dans un plasmide, tel que pTex.

3. Constructions selon la revendication 1 ou 2, **caractérisées en ce qu'**elles codent pour une protéine immunogène de formes promastigotes ou de formes amastigotes de *Leishmania,* ces protéines répondant à l'une des séquences SEQ ID N°6, SEQ ID N°7, SEQ ID -N°9, SEQ ID N°10 et SEQ ID N°12.

4. Protéines immunogènes isolées, **caractérisées en ce qu'**elles présentent une séquence choisie parmi SEQ ID N°6, SEQ ID N°7, SEQ ID N°9, SEQ ID N°10 ou SEQ ID N°12.

5. Souches de *Leishmania* génétiquement modifiées, **caractérisées en ce qu'**elles correspondent à des formes amastigotes ou promastigotes de *Leishmania* transfectées par une construction selon l'une quelconque des revendications 1 à 3.

6. Souches selon la revendication 5, **caractérisées en ce qu'**il s'agit de souches de *L.infantum.*

7. Procédé de transfection d'un parasite de *Leishmania,* **caractérisé en ce qu'**on introduit dans le parasite de *Leishmania* une construction selon l'une quelconque des revendications 1 à 3, comportant un marqueur, on sélectionne les parasites transfectés grâce audit marqueur, on les met en culture dans un milieu axénique et asérique totalement défini et on récupère le surnageant de culture qui renferme les protéines immunogènes présentes dans des concentrations de l'ordre de 10 à 20 fois supérieures à celle produite par la souche mère de *Leishmanie.*

## Claims

1. Nucleic acid constructs, **characterized in that** they comprise isolated nucleic acids in the sense position, capable of encoding an immunogenic protein of promastigote forms or of amastigote forms of *Leishmania,* said nucleic acid corresponding to one of the sequences SEQ ID No 1, SEQ ID No 2, SEQ ID No 3, SEQ ID No 4, SEQ ID No 5 and SEQ ID No 11, said sequences comprising a EcoRV restriction site between the two sites *Sal*I and HindIII or on either side of the *Sal*I site.

2. The constructs of claim 1, wherein said nucleic acid sequences are cloned in the sense position into a plasmid such as pTex.

3. The constructs of claim 1 or 2, **characterized in that** they encode an immunogenic protein of promastigote forms or of amastigote forms of *Leishmania,* these proteins corresponding to one of the sequences SEQ ID No 6, SEQ ID No 7, SEQ ID No 8, SEQ ID No 9, SEQ ID No 10 and SEQ ID No 12.

4. Isolated immunogenic proteins, **characterized in that** they have a sequence chosen from SEQ ID No 6, SEQ ID No 7, SEQ ID No 8, SEQ ID No 9, SEQ ID No 10 or SEQ ID No 12.

5. Genetically modified *Leishmania* strains, **characterized in that** they correspond to *Leishmania* amastigote or promastigote forms transfected with a construct according to any one of claims 1 to 3.

6. Strains according to claim 5, **characterized in that** they are *L. infantum* strains.

7. A method of transfecting a *Leishmania* parasite, **characterized in that** a construct as claimed in any one of claims 1 to 3, comprising a marker, is introduced into the *Leishmania* parasite, the transfected parasites are selected by means of said marker, they are placed in culture in a completely defined axenic and serum-free medium, and the culture supernatant which contains the immunogenic proteins present in concentrations of the order of 10 to 20 times higher than that produced by the *Leishmania* mother strain are recovered.

## Patentansprüche

1. Nucleinsäurekonstrukte, **dadurch gekennzeichnet, dass** sie isolierte Nucleinsäure in Sense-Ausrichtung umfassen, die für ein immunogenes Protein von promastigoten Formen oder amastigoten Formen von *Leishmania* codieren können, wobei die Nucleinsäuren einer der Sequenzen SEQ ID Nr. 1, SEQ ID Nr. 2, SEQ ID Nr. 4, SEQ ID Nr. 5 und SEQ ID Nr. 11 entsprechen, wobei diese Sequenzen eine EcoRV-Restriktionsstelle zwischen der SalI- und der HindIII-Stelle oder beiderseits der SalI-Stelle umfassen.

2. Konstrukte gemäß Anspruch 1, **dadurch gekennzeichnet, dass** die Nucleinsäuresequenzen in Sense-Ausrichtung in ein Plasmid, wie pTex, kloniert sind.

3. Konstrukte gemäß Anspruch 1 oder 2, **dadurch gekennzeichnet, dass** sie für ein immunogenes Protein von promastigoten Formen oder amastigoten Formen von *Leishmania* codieren, wobei diese Proteine einer der Sequenzen SEQ ID Nr. 6, SEQ ID Nr. 7, SEQ ID Nr. 9, SEQ ID Nr. 10 und SEQ ID Nr. 12 entsprechen.

4. Isolierte immunogene Proteine, **dadurch gekennzeichnet, dass** sie eine Sequenz aufweisen, die aus SEQ ID Nr. 6, SEQ ID Nr. 7, SEQ ID Nr. 9, SEQ ID Nr. 10 oder SEQ ID Nr. 12 ausgewählt ist.

5. Genetisch modifizierte *Leishmania*-Stämme, **dadurch gekennzeichnet, dass** sie amastigoten oder promastigoten Formen von *Leishmania* entsprechen, die mit einem Konstrukt gemäß einem der Ansprüche 1 bis 3 transfiziert sind.

6. Stämme gemäß Anspruch 5, **dadurch gekennzeichnet, dass** es sich um Stämme von *L. infantum* handelt.

7. Verfahren zur Transfektion eines Leishmania-Parasiten, **dadurch gekennzeichnet, dass** man in den Leishmania-Parasiten ein Konstrukt gemäß einem der Ansprüche 1 bis 3 einführt, das einen Marker umfasst, die transfizierten Parasiten anhand des Markers selektiert, in einem vollständig definierten axenischen und serumfreien Medium kultiviert und den Kulturüberstand gewinnt, der die immunogenen Proteine enthält, die in Konzentrationen in einer Größenordnung vorhanden sind, die zehn- bis zwanzigmal so groß ist wie die von dem Leishmania-Mutterstamm produzierte.
